# EUROPÄISCHE PATENTANMELDUNG

(11) **EP 1 630 543 A1**
(43) Veröffentlichungstag der Anmeldung: **01.03.2006**
(21) Anmeldenummer: 04104145.0
(22) Anmeldetag: 30.08.2004
(51) Int. Cl.: G01N 21/55, G01N 21/47, G01N 21/59, G01N 33/14

(54) **Sensor zur spektroskopischen Bestimmung gelöster Komponenten in einem fluiden Medium**

(71) Anmelder: Mettler-Toledo GmbH, 8606 Greifensee (CH)
(72) Erfinder: Anders, Klaus-Dieter, 5452, Oberrohrdorf (CH)

(57) **Zusammenfassung**

Ein Sensor ermöglicht die spektroskopische Bestimmung mindestens einer gelösten Komponente, wie z. B. CO₂, in einem fluiden Medium. Dazu transmittiert die Strahlung 8,9 einer Strahlungsquelle 3 durch einen inerten Einsatz 5,11,15 und eine Polymermatrix 6, in der sich die Komponente gelöst hat, wird dann an einem Reflektor 7 reflektiert und zum Detektionssystem 4,4' zurückgeleitet. Der Sensor ist robust gestaltet und in der Lage, den Gehalt einer im Medium gelösten Komponente ohne vorherige Probenentnahme und/oder Konditionierung der Probe zu bestimmen.

## Beschreibung

Die Erfindung betrifft einen Sensor zur spektroskopischen Bestimmung gelöster Komponenten in einem fluiden Medium.

Die Bestimmung bestimmter gelöster Komponenten in einem Medium, vor allem die Bestimmung des Gehalts, wird in den verschiedensten Gebieten, wie z. B. der Getränkeindustrie oder der Biotechnologie, durchgeführt. Beispiele für solche Komponenten sind Kohlendioxid (CO₂), Methanol, Ethanol, Methan sowie andere chemische Substanzen, die in einem fluiden Medium, beispielsweise in einer wässrigen Lösung oder dergleichen, enthalten sind. Vor allem die genaue Kenntnis des CO₂-Gehalts ist für die Getränkeindustrie im Rahmen der Produktionskontrolle von Interesse.

Für die Bestimmung des CO₂-Gehalts listet der Artikel "Selective CO₂ measurement for beverages with the new multiple volume expansion method" in Brauwelt International, (3)2004 verschiedene in der Getränkeindustrie genutzte Verfahren auf, von denen die wichtigsten hier kurz beschrieben werden.

Klassisch wird der CO₂-Gehalt über ein manometrisches Verfahren bestimmt, bei dem der Gesamtdruck oberhalb der Flüssigkeit gemessen und daraus der CO₂₋Gehalt ermittelt wird. Sind auch Fremdgase wie Sauerstoff oder Stickstoff vorhanden, so ergibt dieses Verfahren einen zu hohen CO₂-Gehalt. Der gemessene Wert ist also mit einem Fehler behaftet.

Bei auf der Druck-Temperatur-Messung basierenden Geräten mit einfach expandierender Messkammer wird die Probe in eine entsprechende Kammer eingebracht und dort expandiert, wodurch sich der Einfluss gelöster Fremdgase teilweise reduziert. Eine Weiterentwicklung dieses Verfahrens führt zu einem Mehrfach-Volumen-Expansionsverfahren, bei dem die Probe mehrfach expandiert wird. Auch dieses Verfahren ist noch fehlerbehaftet, da Fremdstoffe bei der Expansion nicht immer vollständig abgetrennt werden oder sich sogar in der Messkammer anlagern können.

Ein weiteres Verfahren basiert auf der Verwendung von permeablen Membranen, deren Materialien auf die zu untersuchende Komponente abgestimmt sind. Dabei bildet die Membran meist ein Fenster einer Kammer, durch das die Komponente in die Kammer eindringt und dann dort als Gas vorliegt. Entweder wird die gasförmige Komponente gleich in dieser Kammer vermessen oder in eine weitere Messkammer überführt. Zur Überführung des Gases wird im Allgemeinen ein Trägergasstrom eingesetzt. Der Gehalt der Komponente in der Probe wird spektroskopisch oder mittels einer Indikatorsubstanz bestimmt. Im Vergleich zu den Druck-Temperatur-Verfahren ergeben sich geringere Messfehler; allerdings kann sich die Gasdurchlässigkeit der Membran im Laufe der Zeit und in Abhängigkeit des Einsatzgebiets ändern, wodurch die Membran an Selektivität verliert. Wird ein Trägergas zum Transport der Komponente verwendet, so wird dem Medium die zu untersuchende Komponente ständig entzogen ohne wieder zurückgeführt zu werden.

Alle bisher vorgestellten Verfahren umfassen eine Probenentnahme oder Konditionierung der Probe. Gerade in fliessenden Medien können sich daher Abweichungen zwischen dem tatsächlichen und dem gemessenen Gehalt ergeben.

Die EP 0253 559 A1 beschreibt eine Vorrichtung und ein Verfahren zur in-vivo-Bestimmung von CO₂ im Blut, wobei die Messung direkt in der Flüssigkeit ohne zusätzliche Messkammer erfolgt. Ein für CO₂ durchlässiges Polymerstück, also eine Polymermatrix, wird mit Strahlung aus dem Infraroten Spektralbereich (lR) bestrahlt und die IR-Absorption des in das Polymer diffundierten Gases gemessen, wobei die Bestrahlung über einen Lichtleiter erfolgt. Für die CO₂-Bestimmung im Blut ist das Polymerstück an einem Lichtleiter befestigt. Die Polymermatrix ist zumindest teilweise mit Metall beschichtet und im Wesentlichen durchlässig für die Wellenlänge des betreffenden spektralen Absorptionsbereichs. Das Polymerstück ist ausserdem permeabel für CO₂ und bildet eine Art Küvette mit einer definierten Weglänge. Der Lichtleiter besteht aus einem CO₂-undurchlässigen Material und leitet sowohl die Strahlung zum Polymerstück als auch die vom Polymerstück reflektierte Strahlung zum Detektor. Für die Messung wird der Lichtleiter in einen flexiblen Katheter eingebaut und über ein Verbindungsstück mit einer schmalbandigen Strahlungsquelle und einem Detektor verbunden. Der CO₂-Gehalt im Polymer wird über die Intensität des IR-Absorptionssignals bei einer Wellenlänge um 4,26 µm bestimmt. Für diese Anwendung muss der Durchmesser des Lichtleiters sehr klein sein, damit auch noch in kleinen Blutgefässen gemessen werden kann. Sowohl der geringe Durchmesser als auch das eingesetzte Material haben zur Folge, dass derartige Lichtleiter leicht brechen. Ferner geht ein Einsatz von Lichtleitern, insbesondere im IR-Spektralbereich, immer mit Intensitätsverlusten einher. Derart aufwändige und empfindliche Systeme sind daher nur geeignet, wenn die Anwendung einen solchen Aufwand erfordert, wie beispielsweise bei kontinuierlichen in-vivo-Blutgasuntersuchungen in sehr kleinen Blutgefässen mittels eines Katheters.

Die Aufgabe der Erfindung ist somit die Bereitstellung eines robusten Sensors zur selektiven Bestimmung des Gehalts einer gelösten Komponente in einem fluiden Medium, wobei der Sensor in der Lage ist, mindestens eine gelöste Komponente abgekoppelt vom Medium, ohne vorherige Probenentnahme und/oder Konditionierung der Probe zu bestimmen.

Gelöst wird diese Aufgabe durch die im Anspruch 1 definierte Vorrichtung.

Ein Sensor zur spektroskopischen Bestimmung mindestens einer in einem fluiden Medium gelösten Komponente, insbesondere von CO₂, umfasst ein Gehäuse, in dem eine Strahlungsquelle, ein Detektionssystem und ein strahlungsdurchlässiges optisches Element angeordnet sind. Ein Teil des Gehäuses wird von dem optischen Element gebildet, welches mit dem Medium in Kontakt steht. Das optische Element umfasst einen inerten Einsatz und eine natürliche oder synthetische Polymermatrix. Als inert werden Stoffe und Materialien bezeichnet, die von Chemikalien nicht angegriffen werden bzw. umgekehrt andere Stoffe chemisch nicht angreifen. Die Polymermatrix weist eine hohe Aufnahmefähigkeit für mindestens eine zu untersuchende Komponente auf. Die von der Strahlungsquelle ausgesandte Strahlung wechselwirkt mit der in der Polymermatrix gelösten Komponente und die zurückgeworfene Strahlung wird von dem Detektionssystem erfasst.

Zwischen der gelösten Komponente im Medium und in der Polymermatrix stellt sich über den Partialdruck der gelösten Komponente ein Gleichgewicht ein. Die Messung erfolgt im Gleichgewicht, daher kann der Sensor auch als Gleichgewichts-Sensor bezeichnet werden. Es besteht ein direkter Zusammenhang zwischen dem von der Polymermatrix aufgenommen Gehalt der Komponente und deren Gehalt im Medium und damit auch zwischen dem gemessenen und dem tatsächlichen Gehalt der Komponente im Medium. Die Polymermatrix ist in der Lage die Komponente sowohl aufzunehmen als auch wieder abzugeben. Der Sensor ermöglicht daher ohne vorherige Probenentnahme und/oder Probenkonditionierung eine sehr genaue und korrekte Messung, ohne dem Medium die Komponente zu entziehen. Die spektroskopische Messung erfolgt abgekoppelt vom Medium und kann daher auch bei trüben und/oder blasenhaltigen Medien angewendet werden. Der Sensor ist kompakt und robust gestaltet, da alle Teile des Sensors sich in einem gemeinsamen Gehäuse befinden und nur wenige optische Bauteile Verwendung finden.

In einigen bevorzugten Ausführungsformen des Sensors wird mindestens ein die Strahlungsquelle und/oder das Detektionssystem umfassender Gehäuse-Bereich von der Polymermatrix durch den inerten Einsatz getrennt. Dadurch wird ein Eindringen der Komponente oder des Mediums in diesen Bereich des Gehäuses verhindert.

Der inerte Einsatz ist vorzugsweise als Stab oder Scheibe ausgebildet. Um eine schnelle Gleichgewichtseinstellung zwischen der Komponente im Medium und in der Polymermatrix zu gewährleisten, steht die Polymermatrix in grossflächigem Kontakt mit dem Medium und weist somit eine grosse Austauschfläche auf.

Bevorzugt ist der Sensor als Transmissions-Sensor oder als ATR-Sensor (ATR; eng. attenuated total reflection) ausgestaltet. Beide Sensor-Arten umfassen einen stabförmigen inerten Einsatz. Der inerte Einsatz des Transmissions-Sensors weist vorzugsweise Aussparungen auf, welche mit einem die Polymermatrix bildenden Polymer gefüllt sind. Der inerte Einsatz des ATR-Sensors hingegen wird vorzugsweise mit der Polymermatrix ummantelt. Der stabförmige inerte Einsatz verbindet bei beiden Sensor-Varianten zwei Bereiche des Gehäuses in denen einerseits die Strahlungsquelle und andererseits das Detektionssystem angeordnet sind.

Vorteilhaft an der Ausführung als ATR-Sensor ist, dass insbesondere ein von der Polymermatrix ummantelter Stab eine sehr grosse Kontaktfläche zwischen Polymermatrix und Medium bereitstellt, so dass sich das Gleichgewicht zwischen den Komponenten im Medium und in der Polymermatrix rasch einstellt.

Ein weiteres bevorzugtes Ausführungsbeispiel ist die Gestaltung des Sensors als Reflektions-Sensor, bei welchem die Strahlungsquelle und das Detektionssystem im gleichen Bereich des Gehäuses angeordnet sind. Damit ein möglichst grosser Anteil der eingebrachten Strahlung wieder das Detektionssystem erreicht, weist das optische Element in einer besonders vorteilhaften Ausgestaltung zusätzlich einen Reflektor auf. Der Reflektor ist vorzugsweise als Metallgitter oder aus Metallpartikeln bestehend gestaltet und befindet sich an der mit dem Medium in Kontakt stehenden Seite der Polymermatrix. Das Reflektormaterial ist korrosions- und oxidationsbeständig. Besonders geeignet sind daher Metalle oder Edelmetalle, insbesondere Edelstahl, Gold, Platin oder Palladium. Damit die mit dem Medium in Kontakt stehende Fläche möglichst glatt ist und Fremdstoffen keine Angriffsfläche zur Anlagerung bietet, wird der Reflektor mit einer dünnen Polymerschicht überzogen oder mit Polymermaterial aufgefüllt. Der Sensor kann bei ausreichender Strahlungsleistung auch ohne Reflektor betrieben werden.

Eine selektive Messung mindestens einer Komponente in einem fluiden Medium ist nur möglich, wenn für die Komponente spezifische Signale detektiert werden. Dies ist der Fall bei der Verwendung von Strahlung aus dem infraroten Spektralbereich (IR). Heteronukleare Komponenten werden durch lR-Strahlung zum Schwingen angeregt und zeigen ein lR-Spektrum mit Schwingungsbanden die charakteristisch für bestimmte chemische Bindungen sind. Beispielsweise weist das lR-Spektrum von CO₂ eine exponierte Bande auf, die nicht von Signalen anderer Komponenten überlagert wird. Diese Bande liegt in einem Wellenlängenbereich um etwa 4,24 µm und eignet sich aufgrund ihrer exponierten Lage für eine selektive CO₂-Gehalts-Bestimmung. Die Intensität der Banden korreliert wie oben stehend erläutert mit dem CO₂-Gehalt in der Polymermatrix und somit auch mit dem CO₂-Gehalt und dem CO₂-Partialdruck im Medium. Die CO₂-Bestimmung erfolgt anhand von spezifischen spektralen Informationen einer in die Polymermatrix eingedrungenen Komponente und somit abgekoppelt vom Medium. Daher ist eine Messung auch in Schwebteilchen oder Bläschen enthaltenden Medien möglich.

Die Polymermatrix wird aus Polymeren gebildet, welche sich neben einer hohen Aufnahmefähigkeit für die aufzunehmende Komponente durch verschiedene andere Eigenschaften auszeichnen. Dazu zählen vor allem die Transparenz für die verwendete Strahlung und die Prozesstauglichkeit, welche eine hohe chemische, thermische und mechanische Beständigkeit erfordert. Bei einem Einsatz in der Getränkeindustrie muss das Polymer auch lebensmittelecht sein. Es sind verschiedene Polymere mit diesen Eigenschaften bekannt, wobei das Polymer vorzugsweise aus der Gruppe bestehend aus Polystyrol, Polyurethan, Polyethylen, Cellulose, Polybutadien, Poly(methylmethacrylat), Polycarbonat, Silikonen, Polymethylpenten (TPX) und Fluorpolymeren, wie Fluorsilikonen, Teflon AF, PFA (Perfluoralkoxy) und FEP (Fluorethylenpropylen), und insbesondere aus der Gruppe bestehend aus Silikonen, Fluorsilikonen, Teflon AF, und TPX gewählt wird. Dabei nimmt von Silikon über TPX zu Teflon AF die Aufnahmefähigkeit für CO₂ ab, die chemische Stabilität der Materialien jedoch zu. In einer bevorzugten Weiterbildung wird die Polymermatrix als Schicht auf dem inerten Einsatz ausgebildet, wobei die Schicht entweder als Polymerfolie oder als Schicht, die aus einem Polymervorläufer direkt auf dem inerten Einsatz auspolymerisiert, aufgebracht wird. Der inerte Einsatz besteht vorzugsweise aus Saphir, einem für lR-Strahlung transparenten Material, welches inert und undurchlässig für die gelösten Komponenten und das Medium ist.

In einer vorteilhaften Ausgestaltung umfasst der Sensor mindestens eine handelsübliche Strahlungsquelle und ein aus mindestens einem handelsüblichen Detektor bestehendes Detektionssystem. Um ein spezifisches Signal mindestens einer zu untersuchenden Komponente zu erfassen, ist die Strahlungsquelle und/oder das Detektionssystem schmalbandig gestaltet, was bewirkt, dass der Einfluss anderer in die Polymermatrix diffundierter Komponenten auf die Bestimmung der zu untersuchenden Komponente minimiert wird. Das Detektionssystem wird insbesondere durch den Einsatz von Filtern schmalbandig gestaltet und ist dafür ausgelegt, mindestens ein Proben- und mindestens ein Referenzsignal gleichzeitig zu empfangen. Das Probensignal ist das komponentenspezifische Signal und das Referenzsignal ist ein lR-Signal , welches weder spektrale Informationen über die zu untersuchende Komponente noch spektrale Informationen über weitere Komponenten enthält.

Bei Verwendung einer geeigneten Kombination aus Strahlenquelle und Detektionssystem können CO₂ und/oder mindestens eine weitere in dem fluiden Medium gelöste Komponente bestimmt werden. Die gelösten Komponenten müssen von der Polymermatrix aufgenommen werden können und spezifische spektroskopische Signale aufweisen. Mögliche weitere Komponenten sind u. a. Kohlenwasserstoffe wie Ethanol, Methanol oder Methan.

Sensoren der beschrieben Art können beispielsweise in bekannte Flansch- oder Tauchsonden eingebaut werden, wodurch sie eine hohe Kompatibilität mit gängigen Prozesssonden und Prozessverfahren aufweisen. Der Sensor umfasst nur wenige optische Bauteile und ist sehr robust, aufgrund der Materialauswahl ist sogar eine relativ aggressive CIP-Reinigung (CIP; eng: clean in place) möglich.

Unterschiedliche Ausführungsformen des Sensors sind stark schematisiert in den Zeichnungen dargestellt, welche im Folgenden beschrieben werden. Es zeigen:
- Fig. 1: eine Darstellung eines bevorzugten Ausführungsbeispiels des Sensors als Reflektions-Sensor im Schnitt;
- Fig. 2: einen als Metallgitter gestalteten Reflektor in einer Polymermatrix im Schnitt;
- Fig. 3: einen aus Metallpartikeln gestalteten Reflektor in einer Polymermatrix im Schnitt;
- Fig. 4: eine Darstellung eines bevorzugten Ausführungsbeispiels des Sensors als Transmissions-Sensor im Schnitt;
- Fig. 5: eine Darstellung eines bevorzugten Ausführungsbeispiels des Sensors als ATR-Sensor im Schnitt;
- Fig. 6: eine Darstellung eines weiteren bevorzugten Ausführungsbeispiels des Sensors als ATR-Reflektions-Sensor im Schnitt.

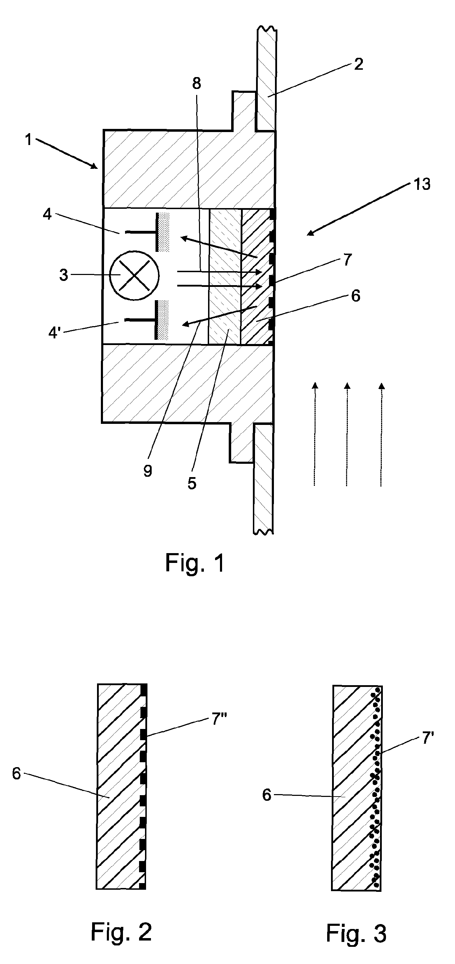

Figur 1 zeigt als erstes Ausführungsbeispiel des Sensors schematisch einen Reflektions-Sensor. Der Sensor ist in einem Gehäuse 1 angeordnet, welches an der Wand 2 eines Behältnisses, z. B. einer Rohrleitung, durch welche das Medium strömt (durch Pfeile angedeutet), befestigt ist. Das Gehäuse 1 ist hier als Teil eines Flansch-Sensors dargestellt. Im Gehäuse 1 befinden sich eine Strahlungsquelle 3, ein Detektionssystem 4, 4' und ein strahlungsdurchlässiges optisches Element 13. Das optische Element 13 weist eine Saphirscheibe 5 als inerten Einsatz, eine Polymermatrix 6 und einen Reflektor 7 auf. Das optische Element 13 bildet dabei den Abschluss des Sensors zum Medium hin. Ein möglicher Strahlengang der lR-Strahlung ist durch die Pfeile 8, 9 lediglich angedeutet. Die Strahlungsquelle 3 und das Detektionssystem 4, 4' befinden sich in einer Art Kammer, die durch die Saphirscheibe 5 begrenzt wird, so dass weder das Medium noch die gelösten Komponenten in die Kammer eindringen können. Die Polymermatrix 6 besteht vorzugsweise aus einem Polymer aus der Gruppe bestehend aus Polystyrol, Polyurethan, Polyethylen, Cellulose, Polybutadien, Poly(methylmethacrylat), Polycarbonat, Silikonen, Polymethylpenten (TPX) und Fluorpolymeren, wie Fluorsilikonen, Teflon AF, PFA (Perfluoralkoxy) und FEP (Fluorethylenpropylen), und insbesondere aus Silikonen, Fluorsilkonen, Teflon AF und TPX. Von Silikon über TPX zu Teflon AF nimmt die Aufnahmefähigkeit für CO₂ ab, die chemische Stabilität der Materialien jedoch zu. Daher werden bevorzugt Silikone eingesetzt. Silikone können als Polymervorläufer direkt auf die Saphirscheibe 5 aufgebracht und anschliessend auspolymerisiert werden. Auf diese Weise haftet die Polymermatrix 6 gut an der Saphirscheibe 5. Zudem wird das Auftreten von Lufteinschlüssen und Unebenheiten vermindert. Der hier als Metallgitter ausgebildete Reflektor 7 wird direkt auf die Polymermatrix 6 aufgebracht und mit einer weiteren dünnen Polymer-Schicht gefüllt oder bedeckt.

Die Strahlungsquelle 3 sendet Strahlung aus dem lR-Bereich aus. Die einfallende Strahlung, durch die Pfeile 8 angedeutet, durchstrahlt die Saphirscheibe 5 und die Polymermatrix 6 und wird am Reflektor 7 diffus reflektiert. Die diffus reflektierte Strahlung, angedeutet durch die Pfeile 9, durchquert ein zweites Mal die Polymermatrix 6 und die Saphirscheibe 5 bevor sie vom Detektionssystem 4, 4' erfasst und an einen Prozessor weitergeleitet wird (hier nicht dargestellt). Der Reflektor 7 dient dazu, einen möglichst grossen Anteil der ausgesandten Strahlung wieder zum Detektionssystem 4, 4' zurückzuführen und dadurch eine ausreichende Signalintensität zu gewährleisten. Das Detektionssystem 4, 4' umfasst in diesem Beispiel zwei mit einem schmalbandigen Filter versehene handelsübliche Detektoren, wie beispielsweise einen PbS-Detektor oder einen pyroelektrischen Detektor. Der Detektor 4 erfasst das Probensignal, welches im Fall von CO₂ im Wellenlängenbereich um etwa 4,24 µm liegt. Der Detektor 4' empfängt als Referenzsignal ein Signal aus einem Bereich des Spektrums an dem sich weder CO₂-Signale noch Signale andere Komponenten befinden, z. B. in einem Wellenlängenbereich um etwa 3,95 µm. Aus diesen beiden Signalen kann mit einer Kalibrierung der CO₂-Partialdruck oder bei Kenntnis der Temperatur des Mediums und der CO₂-Löslichkeit auch der CO₂-Gehalt im Medium ermittelt werden. Die Temperatur wird entweder durch einen in den Sensor integrierten Temperatur-Fühler, z. B. einen Pt100-Widerstand, oder durch andere in die Prozessanlage eingebaute Fühler gemessen. Eine Kalibrierung wird vorab mit Proben mit bekanntem CO₂-Gehalt durchgeführt. Der Sensor wird vorzugsweise werksseitig kalibriert. Je nach Einsatzbedingungen ist es wünschenswert, die Kalibrierung in bestimmten Zeitabständen zu wiederholen, um eine verlässliche Messung zu gewährleisten.

Zwei unterschiedliche Ausführungsformen des Reflektors werden im Folgenden beschrieben. Figur 2 zeigt die Polymerschicht 6 mit Reflektor 7". Der Reflektor 7" ist ein auf die Polymermatrix aufgebrachtes Metallgitter. Dieses ist mit einer dünnen Polymerschicht gefüllt oder überzogen, damit das optische Element 13 (hier nicht dargestellt) eine glatte Kontaktfläche zum Medium aufweist. In Figur 3 ist ebenfalls die Polymermatrix 6 dargestellt. Der Reflektor 7' ist allerdings nicht als Gitter ausgebildet, sondern besteht aus Metallpartikeln, welche in der Polymermatrix eingelagert sind. In einer bevorzugten Weiterbildung befinden sich die Metallpartikel nur in einer dünnen Polymerschicht, welche direkt mit dem Medium in Kontakt steht. Sowohl die Metallpartikel 7' als auch das Metallgitter 7" bestehen aus einem oxidations- und korrosionsbeständigen Material, vorzugsweise einem Metall oder Edelmetall und insbesondere Edelstahl, Gold, Platin oder Palladium.

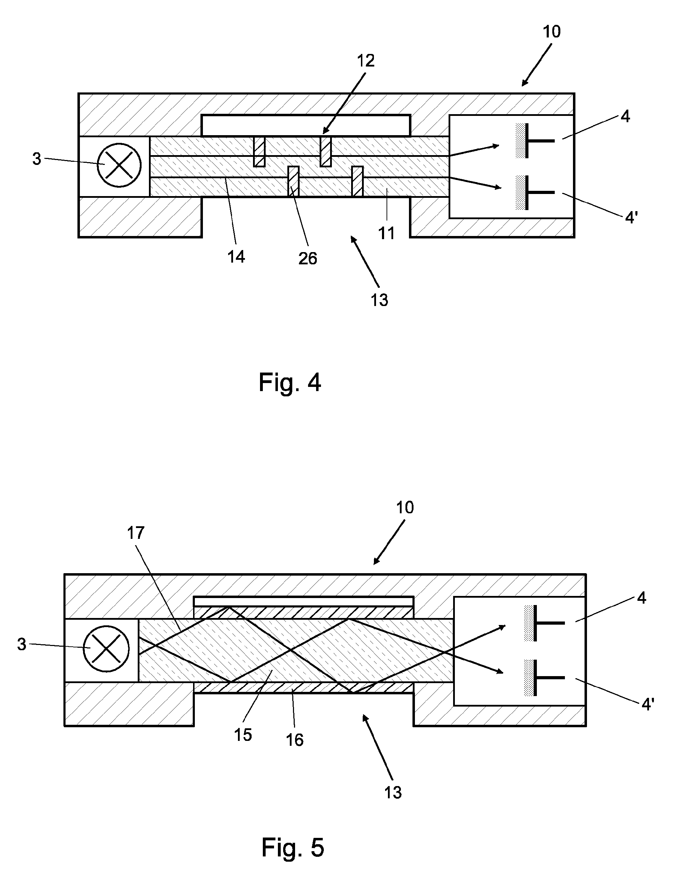

Figur 4 zeigt schematisch als zweites Ausführungsbeispiel des Sensors einen vorzugsweise als Tauchsonde gestalteten Transmissions-Sensor. Der Sensor weist ein Gehäuse 10 auf, das eine Strahlungsquelle 3, ein Detektionssystem 4, 4' und ein optisches Element 13 umfasst. Das Gehäuse 10 wird so in ein das Medium enthaltendes Behältnis eingebracht, dass das optische Element 13 mit dem Medium in direktem Kontakt steht und gleichzeitig einen Teil des Sensor-Gehäuses 10 bildet. Die Strahlungsquelle 3 und das Detektionssystem 4, 4' sind in zwei unterschiedlichen Bereichen des Gehäuses 10 angeordnet, die zumindest durch das optische Element 13 miteinander verbunden sind. Das optische Element 13 besteht in diesem Beispiel aus einem Saphirstab 11 als inertem Einsatz mit mindestens einer Aussparung 12 (vier sind dargestellt). Diese Aussparungen 12 sind mit einem die Polymermatrix 26 bildenden Polymer gefüllt und so gestaltet, dass die Polymermatrix 26 in möglichst grossflächigem Kontakt zum Medium steht. Bei Verwendung von Silikonen, können diese als Polymervorläufer direkt in die Aussparungen 12 eingebracht und dort auspolymerisiert werden. Von der Strahlungsquelle 3 ausgesandte IR-Strahlung, durch die Pfeile 14 angedeutet, durchstrahlt den Saphirstab 11 und die mit der Polymermatrix 26 gefüllten Aussparungen 12. Dabei wechselwirkt die Strahlung mit den in die Polymermatrix 26 hineindiffundierten Komponenten. Die transmittierte Strahlung wird von einem, wiederum aus zwei Detektoren 4, 4' bestehenden Detektionssystem erfasst. Der Detektor 4 empfängt das Probensignal und der Detektor 4' ein Referenzsignal. Wie auch beim ersten Ausführungsbeispiel ist die Strahlungsquelle 3 und/oder das Detektionssystem 4, 4' schmalbandig gestaltet, d. h. mit einem Filter versehen.

Figur 5 zeigt schematisch als drittes bevorzugtes Ausführungsbeispiel einen ATR-Sensor (ATR: attenuated total reflection). Der Aufbau des ATR-Sensors entspricht dem des Transmissions-Sensors aus Figur 4, allerdings mit einem alternativen optischen Element 13, das wiederum mit dem Medium in Kontakt steht und einen Teil des Gehäuses 10 bildet. Das Kernstück dieser Anordnung ist ein von der Polymermatrix 16 umhüllter Saphirstab 15 als optisches Element 13. Hier unterliegt ein kleiner Teil der von der Strahlungsquelle 3 ausgesandten lR-Strahlung (durch die Pfeile 17 angedeutet) einer mehrfachen Totalreflektion an den Grenzflächen des optischen Elements 13. Dabei wechselwirkt die Strahlung mit den in die Polymermatrix 16 diffundierten Komponenten. Erfasst wird bei dieser Sensorform ebenfalls ein Referenz- und ein Probensignal mit einem aus zwei schmalbandigen Detektoren 4, 4' bestehenden Detektionssystem.

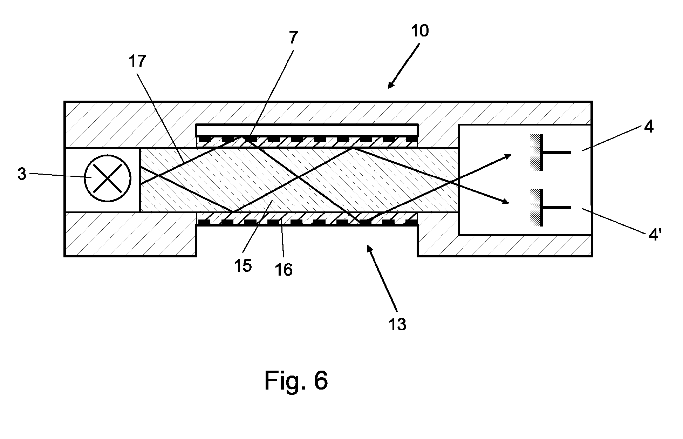

Figur 6 zeigt als besonders vorteilhafte Ausgestaltung einen ATR-Reflektions-Sensor, der im Wesentlichen dem ATR-Sensor entspricht. Bei dieser Sensor-Variante wird das optische Element 13 zusätzlich mit einem Reflektor 7 der oben beschriebenen Art, hier als Metallgitter dargestellt, versehen, um für Einfallswinkel, die nicht zu einer Totalreflektion führen, den Anteil der reflektierten Strahlung und damit die Signalintensität zu erhöhen. Ein solcher Sensor stellt eine Kombination aus einem ATR- und einem Reflektions-Sensor dar.

Der Sensor wurde hauptsächlich für einen Einsatz zur CO₂-Bestimmung beschrieben. Allerdings ist die Verwendung des Sensors nicht darauf beschränkt. Es kann auch der Gehalt anderer gelöster Komponenten, wie beispielsweise Methanol, Ethanol oder Methan, mit diesem Sensor bestimmt werden, solange die verwendete Polymermatrix eine ausreichende Aufnahmefähigkeit für die gelösten Komponenten aufweist, die Komponenten spezifische spektroskopische Signale zeigen und die Strahlenquelle und/oder das Detektionssystem entsprechend gestaltet sind.

Die Bestimmung kohlenwasserstoffhaltiger Komponenten, wie Methanol, Ethanol oder Methan, wird beispielsweise anhand des IR-Signals der CH-Schwingung in einem Wellenlängenbereich um etwa 3,40 µm durchgeführt. Besonders präzise ist diese Bestimmung, wenn das Medium nur eine kohlenwasserstoffhaltige Komponente enthält. Enthält das Medium mehrere kohlenwasserstoffhaltige Komponenten, so wird hauptsächlich der Gesamtgehalt an kohlenwasserstoffhaltigen Komponenten bestimmt.

Alle Ausführungsformen des Sensors sind für einen Einsatz in einer prozesstauglichen Sonde geeignet, können aber selbstverständlich auch in andere Gehäuse eingesetzt werden. Die Integration in eine prozesstaugliche Sonde bedingt eine hohe Kompatibilität mit üblichen Produktionsanlagen.

Der zur Verstärkung der Signalintensität und zur Erhöhung der zum Detektionssystem 4, 4' zurückgeworfenen Strahlung eingesetzte Reflektor 7, 7', 7" kann mit verschiedenen Methoden auf die Polymermatrix 6, 16, 26 aufgebracht werden, dazu gehören neben den beschrieben Methoden der Aufbringung als Metallgitter 7" oder der Einbringung in Form von Metallpartikeln 7' u. a. auch Bedampfen oder Sputtern.

Eine weitere Möglichkeit zur Erhöhung der detektierten Signalintensität stellt ein Auskleiden mindestens eines Bereichs des Gehäuses 1, 10, welcher die Strahlenquelle 3 und/oder das Detektionssystem 4, 4' umfasst, mit einer reflektierenden Schicht dar.

Als Strahlungsquelle 3 wird eine beliebige handelsübliche Strahlungsquelle eingesetzt, die Strahlung im gewünschten spektralen Bereich, vorzugsweise dem infraroten Spektralbereich, insbesondere dem mittleren infraroten Bereich, aussendet. In Abhängigkeit der zu untersuchenden gelösten Komponente kann auch Strahlung aus anderen spektralen Bereichen wie z. B. dem nahem infraroten, dem sichtbaren oder dem ultravioletten Spektralbereich verwendet werden.

Alle oben aufgeführten Ausführungsbeispiele zeigen eine Kombination aus einer breitbandigen Strahlungsquelle 3 mit einem schmalbandigen Detektionssystem 4, 4'. Die Erfindung ist darauf jedoch nicht beschränkt. Es ist auch möglich, die Strahlungsquelle schmalbandig zu gestalten und einen breitbandigen Detektor einzusetzen oder auch beide Bauteile schmalbandig zu gestalten. Die Möglichkeiten zur Gestaltung einer schmalbandigen Strahlungsquelle oder eines schmalbandigen Detektionssystems sind vielfältig. Als ein Beispiel für eine schmalbandige Strahlungsquelle seien hier ein Laser und insbesondere ein Diodenlaser genannt. Eine weitere Möglichkeit besteht darin, eine Breitband-Strahlungsquelle z. B. eine Glowbar oder ein Schwarzkörper-Strahlung aussendendes Glühmittel mit geeigneten Filtern und Aperturen zu versehen. Bezogen auf das Detektionssystem können ebenfalls Filter eingesetzt werden, wobei eine Optimierung der Strahlungsquelle und/oder des Detektionssystems nicht auf die hier beispielhaft angegeben optischen Bauteile begrenzt ist.

Der Sensor ist nicht auf Messungen in einem strömenden Medium beschränkt, sondern, da es sich um einen Gleichgewichts-Sensor handelt, auch in der Lage, den Gehalt einer Komponente in einem stehenden Medium zu bestimmen. Das strömende oder stehende Medium kann sich dabei in einem beliebigen Behältnis befinden. Bei entsprechender Ausgestaltung kann der Sensor auch für die Bestimmung gelöster Komponenten in Gebinden mit kleinen Volumina, wie Flaschen, Messkolben oder anderen kleinen Gefässen und Gebinden eingesetzt werden.

Ebenso stellen die hier angegebenen Polymermaterialien nur eine bevorzugte Auswahl dar. Alle Polymere mit den oben aufgeführten Eigenschaften eignen sich prinzipiell zum Einsatz in derartigen Sensoren.

Der offenbarte Sensor kann sowohl im Labor, z. B. für die Qualitätskontrolle, als auch zur Produktionskontrolle, für die Bestimmung von gelösten Komponenten in fliessenden oder stehenden fluiden Medien, eingesetzt werden.

### Bezugszeichenliste

- 1: Gehäuse
- 2: Wand eines Behältnis
- 3: Strahlungsquelle
- 4, 4': Detektionssystem
- 5: Saphirfenster
- 6: Polymermatrix
- 7: Reflektor
- 7': Reflektor (Metallpartikel)
- 7": Reflektor (Metallgitter)
- 8: einfallende Strahlung
- 9: am Reflektor reflektierte Strahlung
- 10: Gehäuse
- **11**: **Saphirstab mit Aussparungen**
- 12: Aussparung
- 13: optisches Element
- 14: Strahlengang (Transmission)
- 15: Saphirstab
- 16: Polymermatrix
- 17: Strahlengang (ATR)
- 26: Polymermatrix in Aussparung

## Patentansprüche

1. Sensor zur spektroskopischen Bestimmung mindestens einer in einem fluiden Medium gelösten Komponente, insbesondere CO₂, mit einem Gehäuse (1, 10), einer Strahlungsquelle (3), einem Detektionssystem (4, 4') und einem mit dem Medium in Kontakt stehenden strahlungsdurchlässigen optischen Element (13), welches einen inerten Einsatz (5, 11, 15) und eine natürliche oder synthetische Polymermatrix (6, 16, 26), die mindestens eine zu untersuchende Komponente aufnehmen kann und mit dem Medium im Gleichgewicht steht, umfasst, **dadurch gekennzeichnet, dass** die Strahlungsquelle (3), das optische Element (13) und das Detektionssystem (4, 4') im Gehäuse (1, 10) angeordnet sind, wobei das optische Element (13) einen Teil des Gehäuses (1, 10) bildet.

2. Sensor nach Anspruch 1, **dadurch gekennzeichnet, dass** der inerte Einsatz (5, 11, 15) als Stab (11, 15) oder Scheibe (5) ausgeformt und zwischen der Polymermatrix (6, 16, 26) und der Strahlenquelle (3) und/oder dem Detektionssystem (4, 4') angeordnet ist.

3. Sensor nach einem der Ansprüche 1 oder 2, **dadurch gekennzeichnet, dass** dieser als Transmissions- oder als ATR-Sensor ausgebildet ist.

4. Sensor nach einem der Ansprüche 1 bis 4, **dadurch gekennzeichnet, dass** in einem Transmissions-Sensor der inerte Einsatz (11) mindestens eine Aussparung (12) aufweist, in welcher jeweils ein die Polymermatrix (26) bildendes Polymer angeordnet ist.

5. Sensor nach einem der Ansprüche 1 oder 2, **dadurch gekennzeichnet, dass** dieser als Reflektions-Sensor ausgebildet ist, wobei das optische Element (13) einen Reflektor (7, 7', 7") umfasst.

6. Sensor nach Anspruch 5, **dadurch gekennzeichnet, dass** der Reflektor (7, 7', 7") ein auf die Polymermatrix (6, 16, 26) aufgebrachtes Metallgitter (7") ist oder aus in die Polymermatrix (6, 16, 26) eingebrachten Metallpartikeln (7') besteht.

7. Sensor nach Anspruch 6, **dadurch gekennzeichnet, dass** die Metallpartikel (7') oder das Metallgitter (7") aus einem korrosions- und oxidationsbeständigen Metall oder Edelmetall bestehen, insbesondere Edelstahl, Gold, Platin oder Palladium.

8. Sensor nach einem der Ansprüche 1 bis 7, **dadurch gekennzeichnet, dass** die Polymermatrix (6, 16, 26) ein Polymer aus der Gruppe bestehend aus, Polystyrol, Polyurethan, Polyethylen, Cellulose, Polybutadien, Poly(methylmethacrylat), Polycarbonat, Silikonen, Polymethylpenten (TPX) und Fluorpolymeren, wie Fluorsilikonen, Teflon AF, PFA (Perfluoralkoxy) und FEP (Fluorethylenpropylen), und insbesondere aus der Gruppe bestehend aus Silikonen, Fluorsilkonen, Teflon AF und TPX aufweist.

9. Sensor nach einem der Ansprüche 1 bis 8, **dadurch gekennzeichnet, dass** die Polymermatrix (6, 16, 26) aus einer als Polymerfolie aufgebrachten Schicht oder als eine aus einem Polymervorläufer auspolymerisierten Schicht ausgebildet ist.

10. Sensor nach einem der Ansprüche 1 bis 9, **dadurch gekennzeichnet, dass** der inerte Einsatz (5, 11, 15) aus Saphir besteht.

11. Sensor nach einem der Ansprüche 1 bis 10, **dadurch gekennzeichnet, dass** Strahlung aus dem infraroten Spektralbereich (IR) verwendet wird.

12. Sensor nach einem der Ansprüche 1 bis 11, **dadurch gekennzeichnet, dass** die Strahlungsquelle (3) und/oder das Detektionssystem (4, 4') schmalbandig ausgestaltet sind.

13. Sensor nach Anspruch 12, **dadurch gekennzeichnet, dass** die Strahlenquelle (3) und/oder das Detektionssystem (4, 4') mit mindestens einem schmalbandigen Filter versehen sind.

14. Sensor nach einem der Ansprüche 1 bis 13, **dadurch gekennzeichnet, dass** das Detektionssystem (4, 4') für eine Detektion von mindestens einem Proben- und mindestens einem Referenzsignal ausgebildet ist.

15. Sensor nach einem der Ansprüche 1 bis 14 **dadurch gekennzeichnet, dass** der Gehalt von CO₂ und/oder weiterer aus dem fluiden Medium in die Polymermatrix diffundierten Komponenten, insbesondere Ethanol, Methanol oder Methan, bestimmt wird.

16. Sensor nach einem der Ansprüche 1 bis 15, **dadurch gekennzeichnet, dass** der Sensor in einer prozesstaugliche Sonde, insbesondere einer Flansch- oder Tauchsonde, verwendet wird.
